Europäisches Patentamt

European Patent Office

Office européen des brevets

(19)

(11) Veröffentlichungsnummer: **0 451 357 A1**

# (12) EUROPÄISCHE PATENTANMELDUNG

(21) Anmeldenummer: 90124506.8

(22) Anmeldetag: **18.12.90**

(51) Int. Cl.5: **C07G 17/00**, C12N 9/50,
C12N 9/58, A61K 39/00,
A61K 39/395, C12P 21/00,
C12P 21/06, C12N 1/14

(30) Priorität: 13.03.90 DE 4007927

(43) Veröffentlichungstag der Anmeldung:
**16.10.91 Patentblatt 91/42**

(84) Benannte Vertragsstaaten:
**AT BE CH DE DK ES FR GB GR IT LI LU NL SE**

(71) Anmelder: **BAYER AG**

**W-5090 Leverkusen 1 Bayerwerk(DE)**

(72) Erfinder: **Plempel, Manfred, Dr.**
**Zwengenberger Strasse 3c**
**W-5657 Haan(DE)**
Erfinder: **Bremm, Klaus-Dieter, Dr.**
**Vogelsaue 59**
**W-5600 Wuppertal(DE)**
Erfinder: **Gao, Zhan**
**Pahlkestrasse 3**
**W-5600 Wuppertal 1(DE)**

(54) **Pathogenese-Faktoren von Dermatophyten.**

(57) Die vorliegende Erfindung betrifft Pathognese-Faktoren von Dermatophyten, Verfahren zu deren Herstellung und deren Verwendung.

EP 0 451 357 A1

Die vorliegende Erfindung betrifft Pathogenese-Faktoren von Dermatophyten, Verfahren zu deren Herstellung und deren Verwendung.

Dermatophytosen sind Pilzerkrankungen der Haut, die durch Dermatophyten ausgelöst werden.

Derartige Erkrankungen konnten bisher nur mit topisch oder oral wirkenden Medikamenten behandelt werden. Aufgabe der vorliegenden Erfindung war es, neue Möglichkeiten einer Therapie von Dermatophytosen zu finden.

Es wurde nun überraschend gefunden, daß es bei Dermatophyten Pathogenese-Faktoren gibt. Unter Pathogenese-Faktoren werden solche Substanzen (z.B. Proteine) verstanden, die vom Dermatophyten produziert werden und dem Dermatophyten die Infektionen der Haut ermöglichen.

Offenbar werden diese Pathogenese-Faktoren vom Dermatophyten ausgeschieden um dabei zu helfen, Substanzen aus der Umgebung nutritiv zu verwerten. So ist mit den Pathogenese-Faktoren Keratinase-Aktivität assoziiert, möglicherweise haben die Faktoren selbst Keratinase-Aktivität Die Faktoren haben eine Molmasse von mehr als 20 Kili-Dalton (KD).

Dies ergibt sich daraus, daß sie nicht dialysierbar sind, d.h. bei einer Dialyse werden sie von Membranen mit einer Ausschlußgrenze von 20 KD zurückgehalten. Die Produktion der Pathogenese-Faktoren durch die Dermatophyten ist induzierbar. Werden die Dermatophyten mit einem vollständigen Medium (z.B. nach Kimmig oder Sabouraud) kultiviert, entstehen sie nicht bzw. sind nicht nachweisbar. Kultiviert man aber die Dermatophyten in einem Minimal-Medium mit Keratin in Form von Haaren, Haut- oder Nagelmaterial als einziger $N_2$-Quelle (parasitäre Kultur) sind die Faktoren im Kulturüberstand nachweisbar.

Parallel ist eine deutliche Zersetzung des Keratins erkennbar. Interessanterweise sind die Faktoren allein ohne die Dermatophyten in der Lage, das Krankheitsbild einer Dermatophytose auszulösen. Die Faktoren haben antigene Eigenschaften. So sind die Faktoren als Vaccine geeignet, d.h. sie können zur prophylaktischen Immunisierung eingesetzt werden. Derart immunisierte Versuchstiere waren bei einer Infektion vor der Erkrankung geschützt oder zeigten einen wesentlich leichteren Verlauf. Gegen die Faktoren gerichtete Antikörper können auch zur passiven Immunisierung eingesetzt werden. So behandelte Versuchstiere waren ebenfalls von einer Erkrankung geschützt bzw. zeigten einen leichteren Verlauf.

Allgemeines Beispiel

Dermatophyten-Konidien - z.B. von Trichophyton und Microsporum-Arten sowie Epidermophyton floccosum werden in ein Nährmedium eingeimpft, das pro Liter entmineralisiertes Wasser

0,5 g MgSO₄ x 7H₂O, 0,1 g KH₂PO₄
0,01 g FeSO₄ x 7 H₂O und
0,005 g ZnSO₄ x 7 H₂O

enthält und dem als einzige organische Substanz Keratinhaltiges Material wie z.B. Meerschweinchen-, Katzen-, Hunde- oder Menschenhaare, Haut- oder Nagelmaterial in zerkleinerter Form in Mengen zwischen 3 und 10 g/l zugesetzt ist. Die eingeimpften Dermatophyten darin vermögen unter Abbau des Keratinmaterials zu wachsen, vorzugsweise in einem Schüttelinkubator bei 28 - 32 °C.

Nach einer mittleren Kulturdauer von 6-7 Tagen wurden die Kulturen abgenommen und der wäßrige Kulturüberstand vom Pilz-befallenen Keratinmaterial keimfrei abfiltriert. Im Filtrat konnte eine hohe keratolytische Aktivität nachgewiesen werden. Das Filtrat wurde lyophilisiert und zur Entfernung der anorganischen Salze dialysiert.

Gibt man z.B. 0,5 - 1 ml des Dialysates, das die keratolytische Aktivität in hochangereicherter Form enthält, mehrfach pro Tag auf die geschorene Rückenhaut von Meerschweinchen, läßt sich das Krankheitsbild "Dermatophytose" bei diesen Meerschweinchen auslösen, ohne daß lebende Pilzelemente am Entstehen dieser Krankheitsbildes beteiligt sind.

Dies zeigt, daß Dermatophyten-Exkrete an der Symptomatik einer Dermatophytose beteiligt sind.

In einem weiteren Versuch wurden gesunden Meerschweinchen jeden 3.Tag - insgesamt 3 mal - je 0,2 ml des Dialysates subcutan injiziert. Die Tiere wurden 3 Tage nach der letzten Injektion in der üblichen Weise mit dermatophyten Trichophyton mentagrophytes auf der geschorenen Rückenhaut infiziert. Während bei nicht in der geschilderten Weise immunisierten, infizierten Kontrollmeerschweinchen die Dermatophytose in der bekannten Weise ablief, zeigten die immunisierten Tiere keinerlei Infektionszeichen.

Dies zeigt, daß eine Antidermatophytose-Vaccinierung mit dem Pilzexkretionsstoffe (Pathogenese-Faktoren) in angereicherter Form enthaltenden Dialysat möglich ist.

In einem weiteren Experiment wurden Meerschweinchen mit Trich. ment. infiziert. Am 3. Tag p.i., nach dem Auftreten der ersten Infektionszeichen, wurden diesen Tieren je 0,2 ml des Dialysates subcutan injiziert. Schon 2 Tage später zeigten die Infektionsstellen dieser Tiere eine deutliche Heilungstendenz und waren weitere 3 Tage später völlig abgeheilt, während bei den Kontrolltieren die Infektion ihr Maximum erreichte.

Daraus ist abzuleiten, daß die sc-Gabe der im Dialysat oder daraus hergestellter konzentrierter Lösungen in inerten Trägern wie Wasser oder physiologischer Kochsalzlösung enthaltenen Dermatophyten-Antigene, während einer Infektion appliziert, die Antikörperbildung in den infizierten Tieren so stark in Gang setzt, daß ein kurativer Effekt entsteht Die Antikörper können nach bekannten Methoden isoliert werden. Aus den vaccinierten Tieren können auch monoklonale Antikörper nach der von Köhler und Millstein beschriebenen Hybridom-Technik gewonnen werden. Derartige Antikörper können, gegebenenfalls in Arzneimittel-Formulierungen, zur Behandlung von durch Pilzen hervorgerufenen Krankheiten, insbesondere Dermatophytosen, eingesetzt werden.

Beispiel eines Immunisierungsversuches

Es wurden 5 l einer wäßrigen Salzlösung hergestellt, die pro 1 entmineralisiertes Wasser

0,5 g MgSO₄ 7H₂O, 0,1 g KH₂PO₄
0,01 g FeSO₄ 7H₂O und
0,005 g ZuSO₄ 7H₂O

enthielt.

In 250 ml Erlenmeyer-Kolben wurden je 150 ml dieser Lösung abgefüllt und pro Kolben 1 g gewaschene, mit Aceton entfettete und grob zerkleinerte Meerschweinchen-Haare zugesetzt.

Die Kolben wurden 10 Minuten bei 110° C sterilisiert, anschließend mit je 5 x 10⁶ Mikrokonidien einer 3-Wochen-Kultur von Trich. mentagrophytes inokuliert und in einem Schüttelinkubator bei 30° C und 95 UpM für 7 Tage inkubiert.

Die wäßrige Salzlösung aus den Kolben wurde keimfrei abfiltriert und lyophilisiert. Anschließend wurde der trockene Rückstand in insgesamt 50 ml entmineralisiertem Wasser aufgenommen und gegen Wasser für 48 Std. dialysiert.

Das Dialysat wurde in 2 ml Portionen geteilt und eingefroren.

Zur Immunisierung wurden weitere Meerschweinchen am Nacken geschoren und erhielten 3 mal im Abstand von je 3 Tagen pro Tier 0,2 ml des portionsweise aufgetauten Dialysates subcutan injiziert. Die Injektionen wurden gut vertragen.

3 Tage nach der letzten sc-Injektion wurden die Tiere auf dem Rücken geschoren und mit einer angekeimten Mikrokonidien-Suspension von Trich. ment., die pro ml ca 2 x 10⁴ CFU's enthielt, auf einer ca. 3 x 3 cm großen Stelle des Rückens infiziert.

Als Kontrolle dienten Meerschweinchen, die vor der Infektion nicht immunisiert waren. Bei allen diesen Tieren verlief die Dermatophytose normal und erreichte am 12. Tag p.i. ihr Maximum.

Bei den immunisierten Tieren traten bis zu diesem Zeitpunkt keinerlei Infektionszeichen an der Infektionsstelle auf.

**Patentansprüche**

1. Pathogenese-Faktoren von Dermatophyten.

2. Pathogenese-Faktoren gemäß Anspruch 1 mit Keratinase-Aktivität.

3. Pathogenese-Faktoren gemäß den Ansprüchen 1 und 2 mit einer Molmasse von mehr als 10 KD.

4. Vaccine zur Immunisierung gegen Dermatophytosen enthaltend Pathogenese-Faktoren gemäß den Ansprüchen 1 bis 3.

5. Antikörper gegen die Pathogenesefaktoren der Ansprüche 1 bis 3.

6. Arzneimittel enthaltend Antikörper gemäß Anspruch 5.

7. Verfahren zur Herstellung von Pathogenese-Faktoren von Dermatophyten, dadurch gekennzeichnet, daß man Dermatophyten parasitär in Anwesenheit von Keratin kultiviert und anschließend die Faktoren aus dem Kulturüberstand isoliert.

Europäisches Patentamt

**EUROPÄISCHER RECHERCHENBERICHT**

Nummer der Anmeldung

| EINSCHLÄGIGE DOKUMENTE | | | EP 90124506.8 |
|---|---|---|---|
| **Kategorie** | **Kennzeichnung des Dokuments mit Angabe, soweit erforderlich, der maßgeblichen Teile** | **Betrifft Anspruch** | **KLASSIFIKATION DER ANMELDUNG (Int Cl.⁵)** |
| X | CHEMICAL ABSTRACTS, Band 112, Nr. 11, 12. März 1990, Columbus, Ohio, USA R. TSUBOI et al. "Isolation of keratinolytic proteinase from Trichophyton mentagrophytes with enzymic activity at acidic pH" Seite 344, Spalte 2, Zusammenfassung-Nr. 94 316p & Infect. Immun. 1989, 57(11) 3479-83 (Eng.) | 1-3 | C 07 G 17/00 C 12 N 9/50 C 12 N 9/58 A 61 K 39/00 A 61 K 39/395 C 12 P 21/00 C 12 P 21/06 C 12 N 1/14 |
| A | -- | 7 | |
| A | CHEMICAL ABSTRACTS, Band 82, Nr. 13, 31. März 1975, Columbus, Ohio, USA P. DANEW et al. "Peptidase activity of skin-pathogenic fungi. Aminopeptidase activity in the fungus mycelium and culture medium of Microsporum gypseum in relation to the growth phases" Seite 239, Spalte 1, Zusammenfassung-Nr. 82 785q & Mykosen 1974, 17(8), 179-89 (Ger.) -- | 1,2,7 | **RECHERCHIERTE SACHGEBIETE (Int. Cl.⁵)** C 07 G C 12 N 9/00 A 61 K 39/00 C 12 P 21/00 C 12 N 1/00 |
| A | CHEMICAL ABSTRACTS, Band 88, Nr. 3, 16. Januar 1978, Columbus, Ohio, USA T. TANAKA et al. "Formation of histamine and N-acetylhistamine by phatogenic fungi" Seite 296, Spalte 2, Zusammenfassung-Nr. 18 811s & Shinkin to Shinkinsho 1977, | 1 | |

Der vorliegende Recherchenbericht wurde für alle Patentansprüche erstellt.

| Recherchenort | Abschlußdatum der Recherche | Prüfer |
|---|---|---|
| WIEN | 28-06-1991 | SCHNASS |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| | 18(1), 58-64 (Japan.) | | |
| A | CHEMICAL ABSTRACTS, Band 81, Nr. 5, 5. August 1974, Columbus, Ohio, USA B. SInGH et al. "Growth and extracellular lipids of certain pathogenic fungi in presence of different concentrations of amino acids" Seite 94, Spalte 2, Zu-sammenfassung-Nr. 21 568d & Zentralbl. Bakteriol., Parasitenk., Infektionskr. Hyg., Abt. 1 : Orig., Reihe A 1974, 226(2), 278-82 (Eng.) | 1,7 | |
| A | CHEMICAL ABSTRACTS, Band 70, Nr. 7, 17. Februar 1969, Columbus, Ohio, USA V.V. DOBROMYSLOV et al. "Comparative study of free amino acids in pathogenic fungi" Seite 96, Spalte 1, Zu-sammenfassung-Nr. 26 544a & Tr. Leningrad. Khim.-Farm. Inst. 1967, no. 22, 197-203 (Russ.) | 1,7 | RECHERCHIERTE SACHGEBIETE (Int. Cl.⁵) |
| A | AT - B - 346 477 (H. STICKL) * Anspruch 1 * | 4 | |

Der vorliegende Recherchenbericht wurde für alle Patentansprüche erstellt.

| Recherchenort | Abschlußdatum der Recherche | Prüfer |
|---|---|---|
| WIEN | 28-06-1991 | SCHNASS |